# EUROPÄISCHE PATENTSCHRIFT

(11) **EP 0 706 805 B1**
(45) Veröffentlichungstag und Bekanntmachung des Hinweises auf die Patenterteilung: **11.11.1998**
(21) Anmeldenummer: 95115504.3
(22) Anmeldetag: 30.09.1995
(51) Int. Cl.: A61M 5/142, A61N 1/375

(54) **Implantierbares medizinisches Gerät**
Implantable medical device
Dispositif médical implantable

(30) Priorität: 12.10.1994 DE 4436350
(43) Veröffentlichungstag der Anmeldung: 17.04.1996
(73) Patentinhaber: Fresenius AG, 61350 Bad Homburg (DE)
(72) Erfinder: Steinbach, Bernd, D-61347 Bad Homburg v.d.H. (DE)
(74) Vertreter: Luderschmidt, Schüler & Partner GbR

(56) Entgegenhaltungen:
- EP-A- 0 177 250
- EP-A- 0 291 612
- EP-A- 0 347 743
- WO-A-87/04631
- DE-A- 3 241 251

## Beschreibung

Die Erfindung betrifft ein in einen menschlichen oder tierischen Körper implantierbares medizinisches Gerät mit den Merkmalen des Oberbegriffs des Anspruchs 1, wei es beispielsweise aus der EP-A-0 177 250 bekannt ist.

Ein derartiges medizinisches Gerät ist beispielsweise eine implantierbare Infusionspumpe. Derartige Infusionspumpen weisen einen Auslaßkatheter zur Abgabe eines Medikaments an einer gewünschten Stelle im Körper auf. Sie können außerdem eine Fülleitung zum Nachfüllen des Medikaments aufweisen. Durch Bewegung des Implantatträgers bewegen sich die Leitung oder der Katheter relativ zum implantierten Gerät, es wirken Zugkräfte auf die Leitung oder der Katheter, die die Anschlußstellen der Leitung oder des Katheters an dem medizinischen Gerät belasten. Diese Kräfte können so groß sein, daß sich die Leitung oder der Katheter von dem medizinischen Gerät löst. Es kommt dann zu einer unkontrollierten Medikamentenabgabe an einer nicht vorgesehenen Stelle im Körper des Implantatträgers, die ein unkalkulierbares Gesundheitsrisiko in sich birgt.

Ein anderes derartiges medizinisches Gerät ist ein Herzschrittmacher, von dem eine Elektrodenleitung zum Herz des Trägers führt. Auch hier wird die Anschlußstelle der Elektrodenleitung an den Herzschrittmacher infolge von Bewegungen des Implantatträgers belastet, was zum Lösen der Elektrodenleitung führen kann. Dies hat zumindest zur Folge, daß der Herzschrittmacher seine Funktion nicht ausüben kann.

DE 42 21 390 C1 beschreibt einen Portkatheter zur Implantation durch Punktierung. An dem Gehäuse sind Verankerungsschlingen vorgesehen, die sich nach dem Einführen des Katheters abspreizen. Die Anschlußleitung des bekannten Katheters erstreckt sich in axialer Richtung nach außen.

DE-AS 21 24 062 beschreibt eine implantierbare Infusionpumpe, die ein als Strömungsdrossel ausgebildetes Kapillarrohr aufweist. Das Kapillarrohr ist aus Platzgründen nach Art einer Spiralfeder gewickelt. Es umschließt lose einen Rohrstutzen der Infusionspumpe.

Aus der DE-A-32 41 251 ist es bekannt, Herzschrittmacherelektroden beim Einbau zur Erzielung einer Zugentlastung um den Schrittmacher herum in Form einer Schleife zu verlegen.

Die EP-A-0 177 250 beschreibt eine implantierbare Infusionspumpe mit einer an dem Gehäuse befestigten Anschlußleitung, die das Gehäuse teilweise umschlingt.

Aufgabe der Vorliegenden Erfindung ist es, eine Anschlußleitung an einem implantierbaren medizinischen Gerät so anzubringen, daß die Anschlußstelle der Anschlußleitung an das medizinische Gerät wirksam entlastet ist, wenn Zugkräfte auf die Anschlußleitung und Gegenkräfte auf das medizinische Gerät einwirken, um ein Lösen des Anschlusses zu verhindern.

Diese Aufgabe wird erfindungsgemäß durch die Merkmale des Anspruchs 1 gelöst.

Die Anschlußleitung umschlingt das Gerät selbst oder ein eigens dafür vorgesehenes Umschlingungsteil, das am Gerät ein- oder mehrstückig angebracht ist. Eine auf die Anschlußleitung einwirkende Zugkraft wird durch Reibung zwischen dem umschlingenden Teil der Anschlußleitung und dem medizinischen Gerät teilweise oder vollständig auf das medizinische Gerät übertragen, die Anschlußstelle der Anschlußleitung ist teilweise oder vollständig entlastet. Ein Lösen der Anschlußleitung vom medizinischen Gerät wird vermieden. Das Umschlingungsteil kann ein Bolzen oder ein Zapfen sein.

Bereits eine kurze Umschlingung um einen Bruchteil des Umfangs des medizinischen Geräts bzw. um dessen Umschlingungsteil bringt eine Entlastung der Anschlußstelle mit sich. Für eine größere Entlastung der Anschlußstelle wird die Anschlußleitung beispielsweise um den halben Umfang des medizinischen Geräts herumgelegt, oder sie umschlingt das medizinische Gerät ein- oder mehrmals ganz.

Ein von der Anschlußleitung umschlungener Abschnitt des medizinischen Geräts kann beliebige Form haben, also beispielsweise auch eckig sein. Umschlungene Kanten sollten gerundet ausgeführt werden, um eine hohe örtliche Belastung und ggf. ein Zusammenziehen des Querschnitts der Anschlußleitung zu vermeiden. Vorzugsweise ist der Umschlingungsabschnitt rund (nicht unbedingt kreisrund), elliptisch oder oval ausgebildet.

Bei der ersten Ausführungsform der Erfindung ist die Anschlußleitung in eine Nut des medizinischen Geräts eingelegt. Sie befindet sich dadurch an einer festgelegten Stelle des medizinischen Geräts und kann nicht seitlich abrutschen. Die Nut ist so schmal, daß die Anschlußleitung beim Einlegen in die Nut elastisch verformt wird. Insbesondere bei Schlauchleitungen wie Kathetern muß die Verformung so gering sein, daß sich der freie Innenquerschnitt nur gering verkleinert und der Durchlaß gewährleistet ist. Die elastisch verformte Anschlußleitung liegt an Wänden der Nut an, wodurch ebenfalls eine Kraftübertragung von der Anschlußleitung auf das medizinische Gerät bewirkt wird, die die Anschlußstelle zusätzlich entlastet. Der Umschlingungsabschnitt kann dadurch verkürzt werden.

Bei einer zweiten alternativen Ausführungsform der Erfindung weist das medizinische Gerät im Umschlingungsabschnitt eine gezahnte oder gerippte Oberfläche auf, um die Kraftübertragung von der Anschlußleitung auf das medizinische Gerät weiter zu verbessern.

Das medizinische Gerät kann, wie eingangs ausgeführt, ein Herzschrittmacher sein, die Anschlußleitung ist in diesem Fall die elektrisch mit dem Herzschrittmacher verbundene Elektrodenleitung. Ebenso kann das medizinsche Gerät eine implantierbare Infusionspumpe mit einem daran angebrachten Auslaßkatheter sein, der kommunizierend mit einer Medikamentenkammer der Infusionspumpe Verbunden ist.

An der Stelle, an der die Anschlußleitung das medizinische Gerät verläßt und in den Körper des Implantatträgers verlegt ist, ist die Anschlußleitung, beispielsweise mittels eines Bügels oder dgl., an dem medizinischen Gerät gehalten, um zu verhindern, daß an der Anschlußleitung angreifende Zugkräfte über die exzentrisch an das medizinische Gerät herangeführte Anschlußleitung ein Drehmoment auf das medizinische Gerät ausüben und die Anschlußleitung vom medizinischen Gerät abwickeln.

Bei einer bevorzugten Ausgestaltung der Erfindung weist das medizinische Gerät eine Abdeckung auf, unter der der das medizinische Gerät umschlingende Teil der Anschlußleitung verlegt ist.

Nachfolgend wird die Erfindung anhand eines Ausführungsbeispiels näher beschrieben, das in der Zeichnung dargestellt ist. Die einzige Figur zeigt einen Querschnitt durch ein erfindungsgemäßes, implantierbares, medizinisches Gerät.

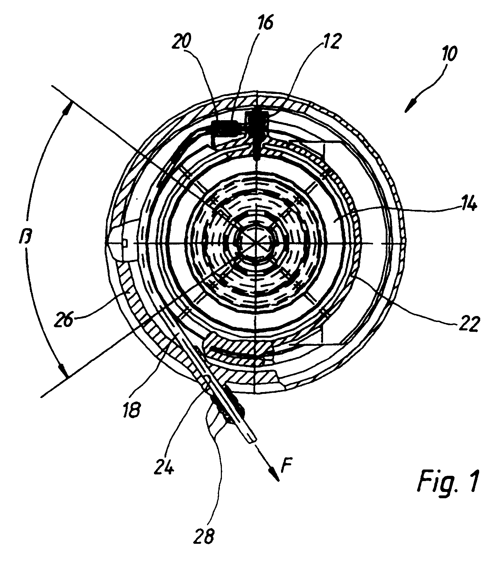

Figur 1 zeigt einen Querschnitt durch eine in einen menschlichen oder tierischen Körper implantierbare Infusionspumpe 10 als Beispiel für ein erfindungsgemäßes medizinisches Gerät. Der Querschnitt der Infusionspumpe 10 ist kreisförmig. Die Infusionspumpe 10 weist an einer Stelle Ihres Umfangs ein Anschlußteil 12 auf, das in eine Medikamentenkammer 14 mündet. An der Außenseite der Medikamentenkammer 14 weist das Anschlußteil 12 einen in etwa tangential stehenden Anschlußstutzen 16 für einen Auslaßkatheter 18 auf. Der Auslaßkatheter 18 ist auf den Anschlußstutzen 16 aufgeschoben und mit einer darübergesteckten Rohrhülse 20 fixiert. Der Auslaßkatheter 18 kommuniziert durch das Anschlußteil 12 hindurch mit der Medikamentenkammer 14.

Zur Zugentlastung der Anschlußstelle des Auslaßkatheters 18 am Anschlußteil 12 der Infusionspumpe 10 ist der Auslaßkatheter 18 um eine zylindrische Wand 22 der Medikamentenkammer 14 herumgelegt und verläßt die Infusionspumpe 10 durch eine als Bohrung ausgeführte Durchführung 24, die in einer Abdeckung 26 angebracht und durch die der Auslaßkatheter 18 hindurchgesteckt ist. Mittels dreier auf den Auslaßkatheter 18 aufgeschobener Ringdichtungen 28 ist dieser an der Durchführung 24 durch die Abdeckung 26 abgedichtet. Die Abdeckung 26 deckt den Auslaßkatheter 18 in dem Bereich ab, in dem letzterer die Wand 22 der Medikamentenkammer 14 umschlingt.

Im ausgeführten Beispiel beträgt der Umschlingungswinkel β 72°. Für eine geringere oder eine stärkere Entlastung der Anschlußstelle kann ein kleinerer oder ein größerer Umschlingungswinkel als im Ausführungsbeispiel, ggf. auch eine mehrfache Umschlingung der Wand 22 vorgesehen werden.

## Patentansprüche

1. Implantierbares medizinisches Gerät mit einer daran befestigten Anschlußleitung (18), die das medizinische Gerät (10) oder einen Teil des medizinischen Geräts (10) zumindest teilweise umschlingt, wobei die Anschlußleitung (18) im Umschlingungsabschnitt (β) an dem medizinischen Gerät (10) oder dessen Teil anliegt, dadurch gekennzeichnet, daß das medizinische Gerät (10) im Bereich der Umschlingung eine Nut aufweist, in die die Anschlußleitung (18) eingelegt ist, wobei die Nut schmaler als eine Querschnittsabmessung der Anschlußleitung quer zur Nut bei unverformter Anschlußleitung ist, oder der Umschlingungsabschnitt (β) des medizinischen Geräts (10) eine gezahnte oder gerippte Oberfläche aufweist.

2. Implantierbares medizinisches Gerät nach Anspruch 1, **dadurch gekennzeichnet,** daß das medizinische Gerät (10) einen in etwa runden Umschlingungsabschnitt (β) aufweist.

3. Implantierbares medizinisches Gerät nach Anspruch 1 oder 2, **dadurch gekennzeichnet,** daß die Anschlußleitung ein Stromkabel ist.

4. Implantierbares medizinisches Gerät nach Anspruch 3, **dadurch gekennzeichnet,** daß das medizinische Gerät ein Herzschrittmacher ist.

5. Implantierbares medizinisches Gerät nach Anspruch 1 oder 2, **dadurch gekennzeichnet,** daß die Anschlußleitung eine Schlauchleitung, insbesondere ein Katheter (18) ist.

6. Implantierbares medizinisches Gerät nach Anspruch 5, **dadurch gekennzeichnet,** daß das medizinische Gerät eine implantierbare Infusionspumpe (10) ist.

7. Implantierbares medizinisches Gerät nach einem der vorhergehenden Ansprüche, **dadurch gekennzeichnet,** daß die Anschlußleitung (18) an der Stelle, an der sie das medizinische Gerät (10) verläßt, durch eine Durchführung (28) hindurch geführt ist.

8. Implantierbares medizinisches Gerät nach einem der vorhergehenden Ansprüche, dadurch gekennzeichnet, daß die Anschlußleitung (18) unter einer Abdeckung (26) des medizinischen Geräts (10) verlegt ist.

## Claims

1. An implantable medical apparatus with a feeder duct (18) fixed thereto which at least partially clasps the medical apparatus (10) or a part of the medical apparatus (10), whereby the feeder duct (18), in the clasping portion (β), rests on the medical apparatus (10) or on a part thereof, characterised in that the medical apparatus (10) has in the clasping portion a groove into which the feeder duct (18) is fitted, the groove being narrower than a cross-sectional dimension of the feeder duct crosswise to the groove in the undeformed state of the feeder duct, or the clasping portion (β) of the medical apparatus (10) has a serrated or ribbed surface.

2. An implantable medical apparatus according to claim 1, characterised in that the medical apparatus (10) has a substantially round clasping portion (β).

3. An implantable medical apparatus according to claim 1 or 2, characterised in that the feeder duct is a power cable.

4. An implantable medical apparatus according to claim 3, characterized in that the medical apparatus is a cardiac pacemaker.

5. Am implantable medical apparatus according to claim 1 or 2, characterised in that the feeder duct is a tubular duct and is in particular a catheter (18).

6. An implantable medical apparatus according to claim 5, characterised in that the medical apparatus is an implantable infusion pump (10).

7. An implantable medical apparatus according to one of the preceding claims, characterised in that the feeder duct (18), at the location at which it leaves the medical apparatus (10), is led through a duct (28).

8. An implantable medical apparatus according to one of the preceding claims, characterised in that the feeder duct (18) is laid under a cover (26) of the medical apparatus (10).

## Revendications

1. Appareil médical implantable comportant un conduit de raccordement (18) fixé à l'appareil et qui s'enroule au moins partiellement autour de l'appareil médical (10) ou d'une partie de l'appareil médical (10), le conduit de raccordement (18) s'appliquant contre l'appareil médical (10) ou une partie de ce dernier dans la section d'enroulement (β), caractérisé en ce que l'appareil médical (10) possède, dans la zone d'enroulement, une gorge, dans laquelle le conduit de raccordement (18) est inséré, la largeur de la gorge étant inférieure à une dimension en coupe transversale du conduit de raccordement transversalement par rapport à la gorge dans le cas où le conduit de raccordement n'est pas déformé, ou bien la section d'enroulement (β) de l'appareil médical (10) possédant une surface dentée ou nervurée.

2. Appareil médical implantable selon la revendication 1, caractérisé en ce que l'appareil médical (10) possède une section d'enroulement (β) approximativement circulaire.

3. Appareil médical implantable selon la revendication 1 ou 2, caractérisé en ce que le conduit de raccordement est un câble d'alimentation en courant.

4. Appareil médical implantable selon la revendication 3, caractérisé en ce que l'appareil médical est un stimulateur cardiaque.

5. Appareil médical implantable selon la revendication 1 ou 2, caractérisé en ce que le conduit de raccordement est un conduit en forme de tuyau, notamment un cathéter (18).

6. Appareil médical implantable selon la revendication 5, caractérisé en ce que l'appareil médical est une pompe à perfusion implantable (10).

7. Appareil médical implantable selon l'une des revendications précédentes, caractérisé en ce que le conduit de raccordement (18) est guidé dans une traversée (28) à l'emplacement où le conduit quitte l'appareil médical (10).

8. Appareil médical implantable selon l'une des revendications précédentes, caractérisé en ce que le conduit de raccordement (18) est disposé sous un capot (26) de l'appareil médical (10).
